(19) Europäisches Patentamt
European Patent Office
Office européen des brevets

(11) EP 2 246 367 A1

(12) **EUROPEAN PATENT APPLICATION**
published in accordance with Art. 153(4) EPC

(43) Date of publication:
**03.11.2010 Bulletin 2010/44**

(21) Application number: **09710895.5**

(22) Date of filing: **13.02.2009**

(51) Int Cl.:
***C08B 37/00*** (2006.01) ***A23K 1/16*** (2006.01)
***A23K 1/18*** (2006.01) ***A23L 1/30*** (2006.01)
***A61K 36/07*** (2006.01) ***A61K 38/00*** (2006.01)
***A61P 31/04*** (2006.01) ***A61P 35/00*** (2006.01)
***C07K 14/375*** (2006.01)

(86) International application number:
**PCT/JP2009/052394**

(87) International publication number:
**WO 2009/102008 (20.08.2009 Gazette 2009/34)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **14.02.2008 JP 2008032935**

(71) Applicant: **Yukiguni Maitake Co., Ltd.**
**Minamiuonuma-shi**
**Niigata 949-6695 (JP)**

(72) Inventors:
- **KODAMA, Noriko**
**Kobe-shi**
**Hyogo 651-0061 (JP)**
- **NANBA, Hiroaki**
**Amagasaki-shi**
**Hyogo 661-0003 (JP)**

(74) Representative: **Schwahn, Hartmut et al**
**Gleiss Grosse Schrell & Partner**
**Patentanwälte Rechtsanwälte**
**Leitzstraße 45**
**70469 Stuttgart (DE)**

# (54) LOW-MOLECULAR-WEIGHT SUBSTANCE DERIVED FROM MAITAKE MUSHROOM AND HAVING IMMUNOSTIMULATING ACTIVITY AND ANTI-TUMOR ACTIVITY

(57) It is an objective of the present invention to develop a Grifola-derived substance having advanced immunopotentiating activity and antitumor activity, which has a molecular weight lower than that of a conventionally known glucan-protein complex and is structurally different from such complex. A Grifola-derived low-molecular-weight substance is produced by the following steps 1) to 4):
1) a step of thermally extracting mycelia or fruit bodies of Grifola with water;
2) a step of adding alcohol to the obtained water-soluble extract fraction to a final concentration between 10% and 80% by volume, allowing the resulting solution to stand at a temperature between 1°C and 40°C, and removing matter floating on or in the solution or adhering to the wall surface of a vessel and a precipitate formed therein;
3) a step of removing alcohol from the water-soluble fraction containing alcohol obtained in step 2) and collecting an adsorbed fraction from the obtained water-soluble fraction by anion-exchange column chromatography;
4) a step of collecting a fraction with a molecular weight of 5000 or less by subjecting the adsorbed fraction to molecular sieving.

EP 2 246 367 A1

## Description

### Technical Field

The present invention relates to a low-molecular-weight protein-glucan complex having advanced immunopotentiating activity and antitumor activity that has been extracted and fractionated from mycelia or fruit bodies of "Maitake" mushroom (Grifola).

### Background Art

A polysaccharide (glucan) having a β-1,6-linked glucose as a main chain and a β-1,3-linked glucose as a branched chain and a polysaccharide (glucan) having a β-1,3-linked glucose as a main chain and a β-1,6-linked glucose as a branched chain extracted from mycelia or fruit bodies of Grifola have been known to have immunopotentiating activity (JP Patent Publication (Kokai) No. 59-210901 A (1984) (Patent Document 1); JP Patent Publication (Kokai) No. 9-238697 A (1997) (Patent Document 2)).
In addition, a method for producing an anticancer substance has been known, wherein "Maitake" (*Grifola frondosa*), "Tonbimaitake" (*Grifola gigantea*), or "Masutake" (*Laetiporus sulphureus*) is extracted with hot water, and the extract is concentrated under reduced pressure so that the resultant is subjected to a combination of a step of precipitation using organic solvents, a step of dialysis for removing low-molecular-weight substances, and a step of removal of impurities by extraction using fat-soluble organic solvents (JP Patent Publication (Kokoku) No. 43-16047 B (1968) (Patent Document 3)).
Examples of glucan-protein complexes obtained by conventional techniques disclosed in Patent Documents 1, 2, and 3 are high-molecular-weight glucan-protein complexes having molecular weights of about 1,000,000. When examining the detailed immunopotentiating activity mechanisms thereof at a cellular level, the fact that such complexes have large molecular weights means that the main active structures thereof cannot be specified. In addition, since such glucan-protein complexes have high molecular weights, they cannot be administered intravenously.
The present inventors previously conducted studies in order to find a low-molecular-weight substance having activity such as immunopotentiating activity, antitumor activity, etc. in a hot water extract of Grifola. As a result, they succeeded in obtaining a glucan-protein complex having a molecular weight of 10,000 to 150,000 (JP Patent Publication (Kokai) No. 2007-31665 A (Patent Document 4)).
However, it is inconvenient to handle such an considerably large glucan-protein complex having a molecular weight of 10,000 to 150,000.

Patent Document 1: JP Patent Publication (Kokai) No. 59-210901 A (1984)
Patent Document 2: JP Patent Publication (Kokai) No. 9-238697 A (1997)
Patent Document 3: JP Patent Publication (Kokoku) No. 43-16047 B (1968)
Patent Document 4: JP Patent Publication (Kokai) No. 2007-31665 A

### Disclosure of the Invention

### Problem to be Solved by the Invention

It is an objective of the present invention to develop a Grifola-derived substance with a low molecular weight having excellent immunopotentiating activity and antitumor activity that is preferably used as a material for pharmaceutical products, foods or drinks, and feeds.

### Means for Solving Problem

The present inventors fractionated a hot water extract of Grifola so as to newly obtain a substance with a molecular weight of 5000 or less comprising a protein-glucan complex (with the protein proportion larger than the glucan proportion) that is structurally different from a conventionally known glucan-protein complex (with the glucan proportion larger than the protein proportion). Then, they found that such substance has activity of promoting *in vitro* growth and proliferation of an established T helper type-1 human cell line (THP-1) and mouse spleen cells. Accordingly, the present inventors completed purification of a substance comprising a low-molecular-weight protein-glucan complex having immunopotentiating activity that is obviously different from substances disclosed in Patent Documents 1, 2, 3, and 4 in terms of molecular weight and protein content.
The present invention relates to the following (1) to (7):

(1) a substance having immunopotentiating activity and antitumor activity containing a protein-glucan complex that exhibits positive results in anthrone reaction and ninhydrin reaction, which is produced by steps comprising the following steps 1) to 4):

1) a step of thermally extracting mycelia or fruit bodies of Grifola with water;
2) a step of adding alcohol to the obtained water-soluble extract fraction to a final concentration between 10% and 80% by volume, allowing the resulting solution to stand at a temperature between 1°C and 40°C, and removing matter floating on or in the solution or adhering to the wall surface of a vessel and a precipitate formed therein;
3) a step of removing alcohol from the obtained water-soluble fraction containing alcohol after step 2) and collecting an adsorbed fraction from the obtained water-soluble fraction by anion-exchange column chromatography;
4) a step of collecting a fraction with a molecular weight of 5000 or less by subjecting the adsorbed fraction to molecular sieving;

(2) the substance having immunopotentiating activity and/or antitumor activity and comprising a protein-glucan complex according to (1), wherein the protein to glucan ratio is 85:15 to 99.5:0.5;
(3) the substance having immunopotentiating activity and/or antitumor activity and comprising a protein-glucan complex according to (1) or (2), wherein the Grifola is "Maitake" (*Grifola frondosa*), "Shiromaitake" (*Grifola albicans Imaz*.), or "Choreimaitake" (*Dendropolyporus umbellatus*);
(4) an immunopotentiating agent or an antitumor agent containing, as an active ingredient, the substance comprising a protein-glucan complex according to any one of (1), (2), and (3);
(5) a chemotherapeutic adjuvant containing, as an active ingredient, the substance comprising a protein-glucan complex according to any one of (1), (2), and (3);
(6) a food or drink containing the substance comprising a protein-glucan complex according to any one of (1), (2), and (3); and
(7) a pet food, a livestock feed, or an additive for a pet food or a livestock feed containing the substance comprising a protein-glucan complex according to any one of (1), (2), and (3).

The present invention is described below in greater detail.
The term "protein-glucan complex" according to the present invention refers to a substance comprising a protein and a glucan. Such substance includes a substance mainly consisting of a protein and having a ratio of protein to glucan of 99.5:0.5 as described in (2) above.
In the present invention, all types of Grifola including "Maitake" (*Grifola frondosa*), "Shiromaitake" (*Grifola albicans Imaz.)*, "Choreimaitake" (*Dendropolyporus umbellatus*), and the like can be used. Fresh Grifola can be used as it is or after cutting it into pieces if necessary. Also, dried Grifola can be used as it is, after cutting it into pieces if necessary, or in a powdered form.
A treatment of thermal extraction with water is carried out at 50°C to 135°C for 15 minutes to 3 hours. For rapid extraction, extraction can be carried out under pressure at 100°C or more, for example, at 1 to 2 atmospheric pressure at about 110°C to 125°C in a pressure pot for 15 to 60 minutes.
As water, distilled water, purified water, ion exchanged water, tap water, and the like are used. About 2 to 50 parts by volume of water are used per part by weight of dried Grifola. When fresh Grifola is used, about 2 to 20 parts by volume of water are used per part by weight of Grifola.
As an alcohol added to an extract obtained by thermal extraction with water, methanol, ethanol, propyl alcohol, and the like can be used. Such alcohol is added to the extract to a final concentration between 10% and 80% by volume. Also, an alcohol with water content between 0% and 50% can be used. After the addition of the alcohol, the resulting solution is allowed to stand for 1 hour to 25 hours, whereby matter floating on or in the solution or adhering to the wall surface of a vessel appears. The matter is then collected from the solution by filtration, pipetting, centrifugation, or straining out with a meshed material.
After the above step, alcohol is removed from the obtained water-soluble fraction containing alcohol. An adsorbed fraction is collected from the obtained water-soluble fraction by anion-exchange column chromatography. For anion-exchange chromatography, generally used anion-exchange resins, such as a strongly basic ion-exchange resin and a weakly basic ion-exchange resin, are used. However, it is preferable to use a highly permeable resin that is chemically and physically stable and has a large ion-exchange volume.
An adsorbed fraction is eluted by a general method and subjected to molecular sieving. Thus, a low-molecular-weight fraction with a molecular weight of 5000 or less can be collected. As a means of molecular sieving, gel filtration chromatography, ultrafiltration, dialysis, or the like can be used. As a gel filtration material, a material widely used for general purposes, such as a dextran-based, agarose-based, cellulose-based, or acrylamide-based material, can be used. Also for ultrafiltration, dialysis, or the like, a variety of apparatuses are commercially available. An adequate apparatus can be used.

It is desirable to select a fraction with a molecular weight of 5000 or less as found by the present inventors. However, the acceptable range of variance in the molecular weight may be ± 20%, depending on apparatus. Within this range, the desired objective can be achieved. In addition, the lower limit of the molecular weight is preferably 500 or greater.

The objective substance of the present invention obtained above is described as follows.

Appearance: an achromatic to yellowish-colored liquid
Solubility: soluble in water, an alcohol solution, an alkaline solution, an acidic solution, and dimethyl sulfoxide
Properties of aqueous solution: neutral to weakly acidic
Color reaction: positive in anthrone reaction and ninhydrin reaction
Molecular weight: 5,000 or less
As a result of purification by column chromatography, the protein to glucan ratio of the substance having immunopotentiating activity and antitumor activity obtained in the present invention was found to range from 85:15 to 99.5:0.5. The ratio would adequately vary depending on the quality of Grifola used as a starting material and conditions for extraction and purification.

The protein-glucan complex of the present invention is a substance having immunopotentiating activity and antitumor activity. Therefore, it may be administered as an immunopotentiating agent or an antitumor agent to humans or animals or may be used in combination with a chemotherapeutic agent such as an antitumor agent or an antibiotic (that is to say, in the form of a chemotherapeutic adjuvant) so as to be administered to humans or animals. In addition, the substance can be added to foods or drinks, pet foods, livestock feeds, and the like.

When the substance of the present invention is used as a pharmaceutical product or an animal drug, it can be prepared in a wide range of dosage forms, since it has a low molecular weight and can be dissolved in water or alcohol. Examples of dosage forms include: solutions, syrups, tablets, granules, powders, capsules, and pills for oral administration; and parenteral injections, drips, suppositories, external preparations, nasal drops, and eye drops for parenteral administration. The substance of the present invention is significantly advantageous for the development of pharmaceutical products.

The substance of the present invention is derived from Grifola, which has been used as a food for many years and therefore is highly safe. Accordingly, it can be used in the form of a food or drink or can be mixed with a food or drink. The term "food or drink" used in the present invention is not limited to drinks such as milk and juice and processed foods that are consumed in daily life. Examples of such foods or drinks include all types of foods, including general foods or drinks such as so-called health foods, foods with health claims such as foods with nutrient function claims and foods for specified health use, supplementary foods for medical use, and special-use foods such as those for elderly person and foods for patients.

## Effects of the Invention

A novel protein-glucan complex with a molecular weight of 5000 or less that was obtained according to the present invention by fractionating a hot water extract of Grifola shows immunopotentiating activity and antitumor activity. It can be used as an immunopotentiating agent, an antitumor agent, or an adjuvant for chemotherapy involving the use of an antibiotic or an antitumor agent.

In addition, such highly safe substance derived from Grifola, which is a very familiar food, can be used not only for pharmaceutical products and animal drugs but also for foods or drinks, feeds, and pet foods. Alternatively, it can be mixed therewith.

This description includes part or all of the contents as disclosed in the description and/or drawings of Japanese Patent Application No. 2008-032935, which is a priority document of the present application.

## Brief Description of the Drawings

Fig. 1 shows the results of a cell proliferation activation test. The cell proliferation activation rate was obtained by the following equation: the cell proliferation activation rate (%) = the absorbance of the treated group (Abs. 450 nm/Abs. 650 nm) / the absorbance of the control group (Abs. 450 nm/Abs. 650 nm) x 100 Fig. 2 shows the amounts ofIL-12 and IFN-γ produced.

Best Mode for Carrying Out the Invention

Hereinafter, the present invention will be specifically described by referring to the following examples.

(1) Extraction Method

Fruit bodies of dried Maitake (*Grifola frondosa*) (150 g) were thermally extracted with 1.5 liters of distilled water at 110°C to 125°C for 15 to 60 minutes. Ethanol was added to 500 ml of the obtained soluble fraction to a final concentration of 10% to 80% by volume. The resulting solution was allowed to stand at 1°C to 40°C for 12 hours or thereabout, whereby viscous brownish matter floating on or in the solution or adhering to the wall surface of a vessel was generated. After collecting the matter by pipetting or the like, a water-soluble fraction containing alcohol obtained by further removing a precipitated substance in a centrifugation step was collected. The water-soluble fraction was obtained from the solution by removing alcohol. Then, the adsorbed fraction was collected by anion-exchange column chromatography (DEAE-Sephadex) therefrom. A fraction having THP-1 cell proliferation activity was obtained by subjecting the solution to gel filtration chromatography (Sephadex or Cellulofine). The substance exhibited positive results in anthrone reaction and ninhydrin reaction. Then, the substance was purified by column chromatography and was found to have a protein to glucan ratio (in a complex) of 85:15 to 99.5:0.5.

As a result of gel filtration chromatography analysis of the molecular weight, the molecular weight was found to be 5,000 or less. The substance obtained by the present invention exhibited ultraviolet absorbance but no visible-light absorbance.

(2) Cell Proliferation Activation Test (*in vitro)*

Mouse spleen cells were cultured with the substance for 18 hours at 37°C under the condition of 5% $CO_2$. Fig. 1 shows the cell proliferation activation rate (%). The group treated with the substance obtained in (1) above exhibited stronger mouse spleen cell proliferation promoting effects than the control group.

(3) Antitumor Test

The substance obtained in (1) above and normal saline solution as a control, respectively, were intraperitoneally administered 7 times to Balb/cA mice, in which colon 26 carcinoma had been implanted, in an amount of 250 μL/kg of body weight. The activity on tumor growth of the substance was examined. The results listed in table 1 were obtained.

[Table 1]

| | Tumor Growth Inhibitory Rate (%) |
|---|---|
| | 8 days after implantation |
| Control Group (treated with normal saline solution) | 0.0 |
| Group treated with the substance | 55.0 |
| (n = 6 mice per group) | |

The tumor growth inhibitory rate was obtained by the following equation:

$$\text{Tumor Growth Inhibitory Rate} = \{1 - (\text{Average Tumor Weight of Treatment Group (g)} / \text{Average Tumor Weight of Control Group (g)})\} \times 100.$$

As is apparent from table 1, stronger tumor growth inhibitory effects were exhibited in the group to which the substance obtained in (1) above had been administered compared with those exhibited in the control group. In addition, to examine the activation of immunocompetent cells in tumor-bearing mice, spleen cells were cultured for 24 hours at 37°C under the condition of 5% $CO_2$. Fig. 2 shows the amounts of IL-12 and IFN-γ produced. Based on the results of the cell proliferation activation test in (2) above and the antitumor test in (3) above, it was confirmed that the substance obtained in the present invention has strong immunopotentiating activity and antitumor activity.

All publications, patents, and patent applications cited herein are incorporated herein by reference in their entirety.

## Claims

1. A substance having immunopotentiating activity and antitumor activity containing a protein-glucan complex that exhibits positive results in anthrone reaction and ninhydrin reaction, which is produced by steps comprising the following steps 1) to 4):

    1) a step of thermally extracting mycelia or fruit bodies of Grifola with water;
    2) a step of adding alcohol to the obtained water-soluble extract fraction to a final concentration between 10% and 80% by volume, allowing the resulting solution to stand at a temperature between 1°C and 40°C, and removing matter floating on or in the solution or adhering to the wall surface of a vessel and a precipitate formed therein;
    3) a step of removing alcohol from the obtained water-soluble fraction containing alcohol after step 2) and collecting an adsorbed fraction from the obtained water-soluble fraction by anion-exchange column chromatography;
    4) a step of collecting a fraction with a molecular weight of 5000 or less by subjecting the adsorbed fraction to molecular sieving.

2. The substance having immunopotentiating activity and/or antitumor activity and comprising a protein-glucan complex according to claim 1, wherein the protein to glucan ratio is 85:15 to 99.5:0.5.

3. The substance having immunopotentiating activity and/or antitumor activity and comprising a protein-glucan complex according to claim 1 or 2, wherein the Grifola is "Maitake" (*Grifola frondosa*), "Shiromaitake" (*Grifola albicans Imaz*.), or "Choreimaitake" (*Dendropolyporus umbellatus*).

4. An immunopotentiating agent or an antitumor agent containing, as an active ingredient, the substance comprising a protein-glucan complex according to any one of claims 1, 2, and 3.

5. A chemotherapeutic adjuvant containing, as an active ingredient, the substance comprising a protein-glucan complex according to any one of claims 1, 2, and 3.

6. A food or drink containing the substance comprising a protein-glucan complex according to any one of claims 1, 2, and 3.

7. A pet food, a livestock feed, or an additive for a pet food or a livestock feed containing the substance comprising a protein-glucan complex according to any one of claims 1, 2, and 3.

Fig. 1

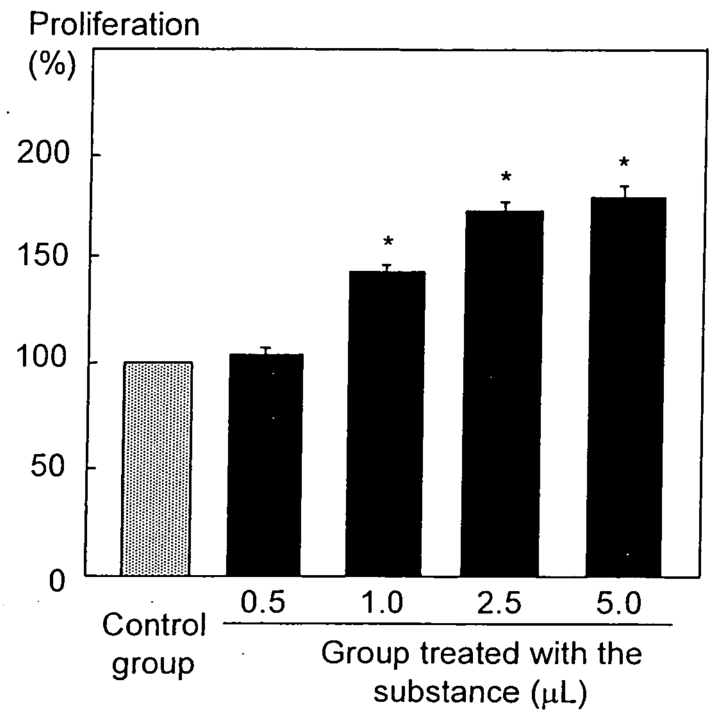


Mean ± SE (n = 10): *Scheffe's *t* test (significant difference confirmed within 5%)

Fig. 2

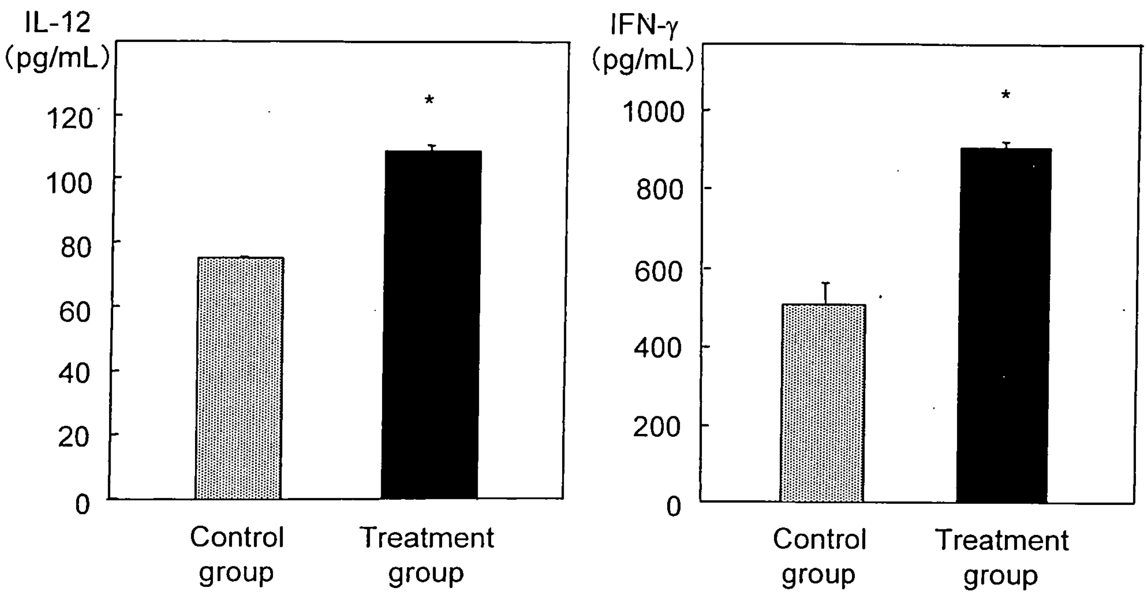


Mean ± SE (n = 3): *Student's *t* test (significant difference confirmed within 5%)

**INTERNATIONAL SEARCH REPORT**

International application No.
PCT/JP2009/052394

A. CLASSIFICATION OF SUBJECT MATTER
*C08B37/00*(2006.01)i, *A23K1/16*(2006.01)i, *A23K1/18*(2006.01)i, *A23L1/30*(2006.01)i, *A61K36/07*(2006.01)i, *A61K38/00*(2006.01)i, *A61P31/04*(2006.01)i, *A61P35/00*(2006.01)i, *C07K14/375*(2006.01)i

According to International Patent Classification (IPC) or to both national classification and IPC

B. FIELDS SEARCHED

Minimum documentation searched (classification system followed by classification symbols)
C08B37/00, A61K36/07, A61K38/14, A61K38/16, A23K1/16-A23K1/18, A23L1/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
WPI

C. DOCUMENTS CONSIDERED TO BE RELEVANT

| Category* | Citation of document, with indication, where appropriate, of the relevant passages | Relevant to claim No. |
|---|---|---|
| X<br>Y | JP 43-16047 B1 (Kureha Chemical Industry Co., Ltd.),<br>06 July, 1968 (06.07.68),<br>(Family: none) | 6,7<br>1-5 |
| X<br>Y | JP 63-56881 B2 (The Mushroom Research Institute of Japan),<br>09 November, 1988 (09.11.88),<br>(Family: none) | 6,7<br>1-5 |
| X<br>Y | JP 2007-31665 A (Yukiguni Maitake Corp.),<br>08 February, 2007 (08.02.07),<br>& US 2007/0027069 A1 | 6,7<br>1-5 |
| X<br>Y | JP 55-159796 A (Amano Enzyme Inc.),<br>12 December, 1980 (12.12.80),<br>(Family: none) | 6,7<br>1-5 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 May, 2009 (01.05.09) | 19 May, 2009 (19.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 59210901 A
- JP 9238697 A
- JP 4316047 B
- JP 2007031665 A
- JP 2008032935 A